(19) Europäisches Patentamt
European Patent Office
Office européen des brevets

(11) Veröffentlichungsnummer: **0 005 172**
**B1**

(12) # EUROPÄISCHE PATENTSCHRIFT

(45) Veröffentlichungstag der Patentschrift: **29.04.81**

(51) Int. Cl.³: **C 07 D 405/04**, D 06 L 3/12

(21) Anmeldenummer: **79100990.5**

(22) Anmeldetag: **31.03.79**

(54) **Cumarin-Verbindungen und ihre Verwendung als Aufheller für organische hochmolekulare Materialien.**

(30) Priorität: **13.04.78 DE 2816028**

(43) Veröffentlichungstag der Anmeldung: **14.11.79 Patentblatt 79/23**

(45) Bekanntmachung des Hinweises auf die Patenterteilung: **29.04.81 Patentblatt 81/17**

(84) Benannte Vertragsstaaten: **BE CH DE FR GB IT**

(56) Entgegenhaltungen:
**DE-A-1 695 817**
**DE-A-2 161 343**
**DE-A-2 712 408**
**DE-B-1 694 362**

(73) Patentinhaber: **BAYER AG, Zentralbereich Patente, Marken und Lizenzen, D-5090 Leverkusen 1, Bayerwerk (DE)**

(72) Erfinder. **Dorlars, Alfons, Dr., Mozartstrasse 32, D-5090 Leverkusen (DE)**
Erfinder: **Neuner, Otto, Dr., Heiligenstock 77, D-5060 Bergisch-Gladbach 2 (DE)**
Erfinder: **Schellhammer, Carl-Wolfgang, Dr., Katharinental 26, D-5060 Bergisch-Gladbach 2 (DE)**
Erfinder: **Schroeder, Josef, Dr., Paul-Klee-Strasse 64, D-5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

EP 0 005 172 B1

BUNDESDRUCKEREI BERLIN

# Cumarin-Verbindungen und ihre Verwendung als Aufheller für organische hochmolekulare Materialien

Die Erfindung betrifft Cumarin-Verbindungen der Formel

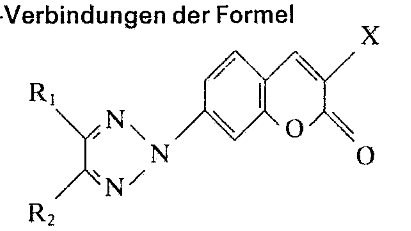

(I)

worin

$R_1$ und $R_2$ für Wasserstoff, $C_1-C_6$-Alkyl, das durch Hydroxy, $C_1-C_4$-Alkoxy, Cyan, Carboxy, Sulfo, ($C_1-C_4$-Alkoxy)-carbonyl, Amino, Mono- und Di-($C_1-C_4$-alkyl)-amino, Aminocarbonyl, Mono- und Di-($C_1-C_4$-alkyl)-aminocarbonyl, Aminosulfonyl, Mono- und Di-($C_1-C_4$-alkyl)-aminosulfonyl, $C_1-C_4$-Alkylthio, Halogen, vorzugsweise Fluor, Chlor und Brom, $C_1-C_4$-Alkylsulfonyl und Phenyl substituiert sein kann;
Phenyl, das durch $C_1-C_4$-Alkyl, Trifluormethyl, Chlor, Brom, Cyan, ($C_1-C_4$-Alkoxy)-carbonyl, $C_1-C_4$-Alkoxy und Phenyl substituiert sein kann;
Phenyl-$C_1-C_4$-alkyl, das im Phenylkern noch durch Chlor, $C_1-C_4$-Alkyl und $C_1-C_4$-Alkoxy substituiert sein kann, stehen, wobei

$R_1$ und $R_2$ jedoch nicht gleichzeitig Wasserstoff bedeuten können und

X Cyan oder $-COOR_3$ bedeutet, wobei

$R_3$ für $C_1-C_6$-Alkyl, das durch Hydroxy, $C_1-C_4$-Alkoxy, Cyan, Carboxy, Sulfo, ($C_1-C_4$-Alkoxy)-carbonyl, Amino, Mono- und Di-($C_1-C_4$-alkyl)-amino, Aminocarbonyl, Mono- und Di-($C_1-C_4$-alkyl)-aminocarbonyl, Aminosulfonyl, Mono- und Di-($C_1-C_4$-alkyl)-aminosulfonyl, $C_1-C_4$-Alkylthio, Halogen, vorzugsweise Fluor, Chlor und Brom, $C_1-C_4$-Alkylsulfonyl und Phenyl substituiert sein kann, stehen.

Zur Synthese der Verbindungen der Formel (I) stehen verschiedene Wege zur Verfügung:

A. Man kann 7-Hydrazino-cumarinverbindungen II mit $\alpha$-Isonitrosoketonen III zu den Hydrazonen IV kondensieren, die bei der wie üblich durchgeführten Cyclisierung die 7-[1,2,3-Triazolyl-(2)]-cumarinverbindungen I ergeben.

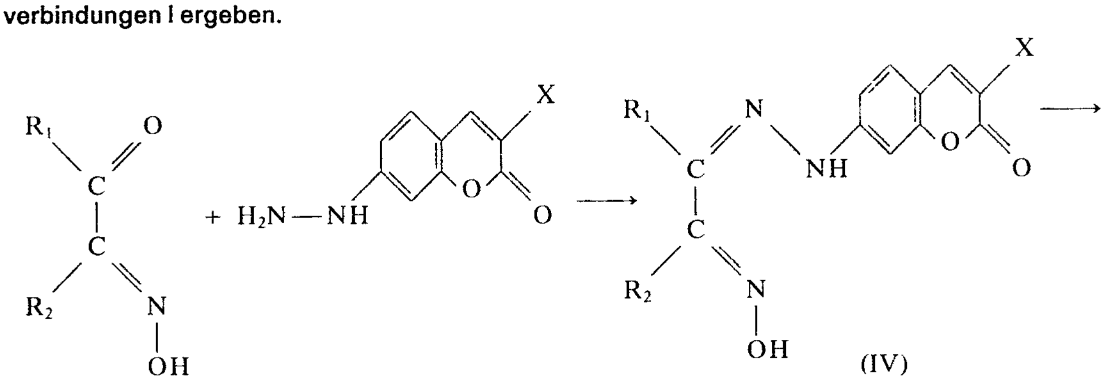

(I)

(III) (II) (IV)

B. Die Kondensation von 4-[1,2,3-Triazolyl-(2)]-salicylaldehyden V mit Malonsäuredialkylestern oder Cyanessigestern VI in Gegenwart von basischen Katalysatoren liefert ebenfalls die 7-[1,2,3-Triazolyl-(2)]-cumarinverbindungen I.

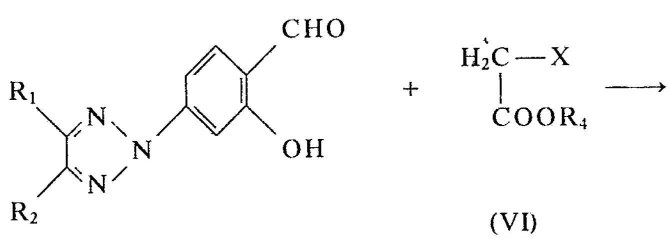

(I)

(V) (VI)

In Formel VI bedeutet $R_4$ einen Alkylrest, bevorzugt den Methyl- oder Äthylrest.

C. Man kann 7-[1,2,3-Triazolyl-(2)]-cumarin-3-carbonsäure VII mit einem passenden Alkohol $R_3OH$ direkt oder nach Überführung in das Säurechlorid VIII zu Verbindungen der Formel Ia umsetzen.

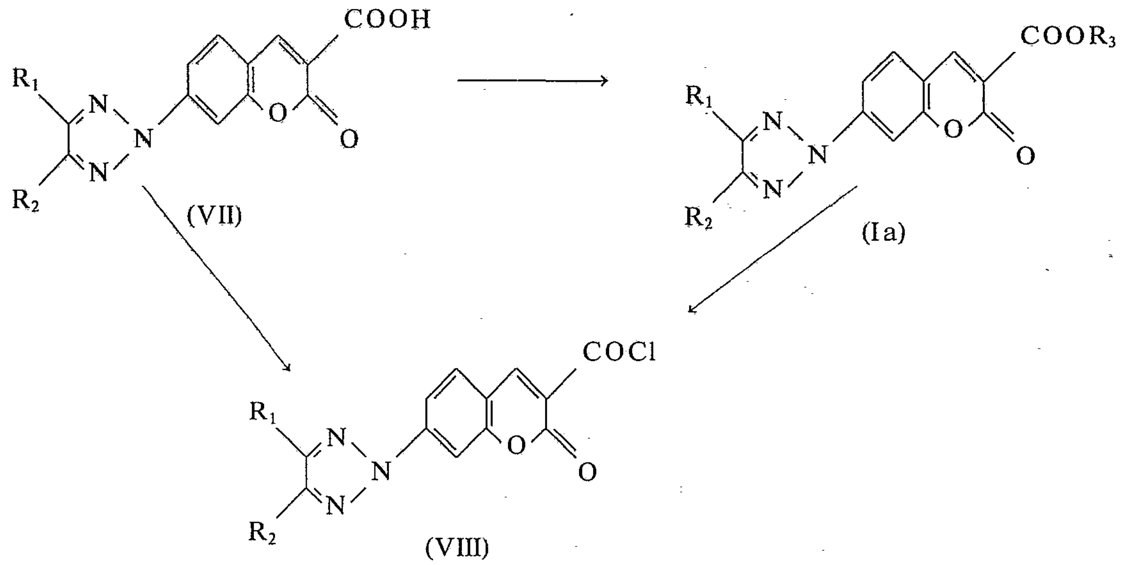

(VII) (Ia) (VIII)

D. Bei der Kupplung von 3-Carboalkoxy- oder 3-Cyano-cumarin-7-diazoniumsalzen IX auf in 3- und 4-Stellung substituierte Isoxazolin-5-one entstehen Azofarbstoffe X, die beim Erhitzen in Gegenwart von tertiären Aminen und von sekundären Alkoholen unter Verlust von $CO_2$ in 7-[1,2,3-Triazolyl-(2)]-cumarinverbindungen I übergehen.

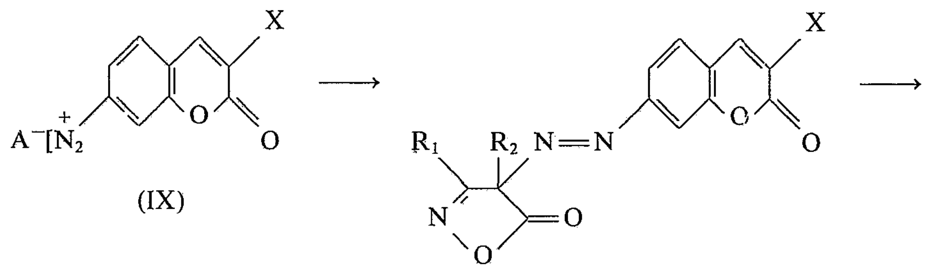

(IX) (I)

Die Ausgangsverbindungen der Formeln II, III, V, VI, VII und IX sind literaturbekannt oder nach Analogverfahren herstellbar.

Die erfindungsgemäßen Verbindungen der Formel I können als Weißtöner Verwendung finden. Sie eignen sich zum Weißtönen von synthetischen, halbsynthetischen und natürlichen organischen hochmolekularen Materialien, insbesondere zum Aufhellen von Fasern, Fäden, Geweben, Gewirken oder Folien synthetischer Herkunft, bevorzugt zum Aufhellen von Polyäthylenglykolterephthalat. Sie können in üblicher Weise angewendet werden, z. B. in Form von wäßrigen Dispersionen oder in Form von Lösungen in indifferenten Lösungsmitteln. Die jeweils erforderlichen Mengen an Weißtöner liegen im allgemeinen unter 0,5%, bezogen auf das Gewicht des zu behandelnden Materials.

Die neuen Verbindungen liefern eine hervorragende Aufhellwirkung auf den genannten Substraten. Die erhaltenen Aufhellungen sind lichtecht und waschbeständig.

Diese Effekte werden von ähnlichen, aus DE-AS 1 694 362, DE-OS 1 695 817, DE-OS 2 161 343 und DE-OS 2 712 408 bekannten Cumarinverbindungen nicht im gleichen Maße erzielt.

So ist etwa der aus Beispiel 10 der DE-AS 1 694 362 bekannte Aufheller konstitutionell nächstvergleichbaren erfindungsgemäßen Verbindungen hinsichtlich des Weißeffektes von Polyester-HT-Färbungen unterlegen.

Dieses Ergebnis muß als ausgesprochen überraschend bezeichnet werden, da es in keiner Weise vorhersehbar war.

## Beispiel 1

Ein Gewebe aus Polyäthylenglykolterephthalat wird im Flottenverhältnis 1 : 40 in ein Bad eingebracht, welches im Liter 1,5 g Natriumoleylsulfat, 0,75 g Ameisensäure und 0,05 g 7-[4-Methyl-5-phenyl-1,2,3-triazolyl-(2)]-3-carbäthoxy-cumarin enthält. Das Bad wird in 30 Minuten auf 97° C erhitzt

und 45 Minuten bei dieser Temperatur gehalten, wobei das Gewebe mäßig bewegt wird. Anschließend wird es gespült und getrocknet. Es besitzt dann eine hervorragende, rotstichige Aufhellung.

Der verwendete Weißtöner wurde wie folgt hergestellt: 27,5 g 7-Acetylamino-3-carbäthoxy-cumarin (F. 243–244° C) werden in 250 ml Äthanol unter Zugabe von 50 ml konz. Salzsäure 6 Stunden zum Sieden erhitzt, abgekühlt, das Aminhydrochlorid abgesaugt und mit wenig Äthanol gewaschen. Das Festprodukt wird in einer Mischung aus 150 ml Wasser, 30 g Natriumchlorid und 6 ml konz. Schwefelsäure bei 0 bis 10° C mit einer wäßrigen Natriumnitritlösung diazotiert und in bekannter Weise mit Zinn(II)-chlorid reduziert.

28,2 g 7-Hydrazino-3-carbäthoxy-cumarin (F. 185–186° C) werden in 150 ml Äthylenglykolmonomethyläther in Gegenwart von 10 ml 50%iger Essigsäure bei 70° C gelöst, mit 28,4 g Oximinopropiophenon versetzt und insgesamt 6 Stunden bei 90 bis 100° C gerührt. Das auskristallisierte Oximinohydrazon wird abgesaugt und getrocknet.

32 g Oximinohydrazon vom F. 247–249° C werden in 150 ml Äthylenglykolmonomethylätheracetat in Gegenwart von 8 g wasserfreiem Natriumacetat mit 10 ml Essigsäureanhydrid 3 Stunden bei 80° C und 1 Stunde bei 120° C verrührt. Das Beim Abkühlen auskristallisierende Triazol schmilzt nach dem Umlösen aus Dioxan bei 181 bis 184° C. Ausbeute 20 g.

## Beispiel 2

Ein ähnliches Färbeergebnis wie in Beispiel 1 erhält man, wenn man anstelle des dort genannten Weißtöners 7-[4-Äthyl-5-methyl-1,2,3-triazolyl-(2)]-3-carbäthoxy-cumarin verwendet. Zur Synthese dieser Verbindung werden 23,1 g 4-[4-Äthyl-5-methyl-1,2,3-triazolyl-(2)]-salicyclaldehyd, 19,2 g Malonsäurediäthylester, 2 ml Piperidin und 60 ml Äthanol 5 Stunden rückfließend gekocht. Das nach dem Abkühlen isolierte Kristallisat wird aus Toluol umgelöst; man erhält 22,9 g gelbe Kristalle vom F. 159° C.

## Beispiel 3

Polyäthylenglykolterephthalatgewebe wird in einem Autoklaven im Flottenverhältnis 1 : 25 in einem Bade folgender Zusammensetzung behandelt: 0,2% (bezogen auf das Fasergewicht des aufzuhellenden Gewebes) 7-[4-Methyl-5-phenyl-1,2,3-triazolyl-(2)]-3-carbomethoxy-cumarin in fein dispergierter Form, 1 g eines äthoxylierten Stearylalkohols, 1000 ml enthärtetes Wasser. Man erhitzt die Mischung im Verlaufe von 30 Minuten von 40 auf 120° C, beläßt sie weitere 30 Minuten bei 120° C, kühlt ab, spült und trocknet. Man erhält so ein Polyestergewebe von brillantem, rotstichigem Weiß.

Zur Herstellung von 7-[4-Methyl-5-phenyl-1,2,3-triazolyl-(2)]-3-carbomethoxy-cumarin überführt man 7-[4-Methyl-5-phenyl-1,2,3-triazolyl-(2)]-cumarin-3-carbonsäure mit Thionylchlorid in das Säurechlorid vom F. 198° C, das beim Kochen mit Methanol den im Beispiel genannten Methylester vom F. 178° C ergibt.

## Beispiele 4 und 5

Mit ähnlich gutem Aufhellerfolg wie in Beispiel 3 können 7-[4-Methyl-5-phenyl-1,2,3-triazolyl-(2)]-cumarin-3-carbonsäure-β-methoxyäthylester vom F. 145° C oder 7-[4-Methyl-5-phenyl-1,2,3-triazolyl-(2)]-cumarin-3-carbonsäure-butylester vom F. 147° C verwendet werden.

## Beispiel 6

Ein Gewebe aus Polyäthylenglykolterephthalat wird mit einer wäßrigen Suspension, die im Liter 1 g 7-[4-Methyl-5-phenyl-1,2,3-triazolyl-(2)]-3-carbäthoxy-cumarin, 2 g handelsübliches Dispergiermittel und 1 g handelsübliches Netzmittel enthält, geklotzt. Das Gewebe wird dann auf eine Gewichtszunahme von 100% abgequetscht, bei 60° C getrocknet und 40 Sekunden auf 170° C erhitzt. Das so behandelte Gewebe zeigt gegenüber unbehandelter Ware eine starke, rotstichige Aufhellung von guter Lichtbeständigkeit.

Beispiele 7 bis 11

Aufhellungen mit guten Effekten und Echtheiten erhält man auch, wenn man gemäß Beispiel 6 das Polyäthylenglykolterephthalat-Gewebe mit folgenden Verbindungen behandelt:

7-[4-Phenyl-1,2,3-triazolyl-(2)]-3-carbäthoxy-cumarin vom F. 196° C,
7-[4-Biphenylyl-5-methyl-1,2,3-triazolyl-(2)]-3-carbäthoxy-cumarin vom F. 240° C,
7-[4-Methyl-5-phenyl-1,2,3-triazolyl-(2)]-3-cyano-cumarin vom F. 223 – 227° C,
7-[4-Phenyl-1,2,3-triazolyl-(2)]-3-cyano-cumarin vom F. 285-287° C oder
7-[4-Äthyl-5-methyl-1,2,3-triazolyl-(2)]-3-cyano-cumarin vom F. 253 – 256° C.

Beispiel 12

Polyacrylnitrilfasern werden im Flottenverhältnis 1 : 40 in ein wäßriges Bad eingebracht, das im Liter 1 g Oxalsäure, 1 g Natriumchlorit sowie 0,05 g 7-[4-Methyl-5-phenyl-1,2,3-triazolyl-(2)]-3-carbäthoxy-cumarin enthält. Das Bad wird innerhalb von 20 Minuten auf 98° C erhitzt und 60 Minuten bei dieser Temperatur gehalten. Anschließend wird das weißzutönende Gut gespült und getrocknet, es ist dann sehr schön rotstichig aufgehellt.

Beispiel 13

Ein Gewebe aus Celluloseacetatfasern wird mit einer Lösung von 1 g 7-[4-Benzyl-5-phenyl-1,2,3-triazolyl-(2)]-3-carbäthoxy-cumarin in einem Liter Perchloräthylen geklotzt. Man quetscht dann auf eine Gewichtszunahme von 70% ab, trocknet bei 50° C und fixiert dann den Weißtöner durch Erhitzen auf 190° C während 20 Sekunden. Das Gewebe ist dann hervorragend rotstichig aufgehellt. Das verwendete Aufhellungsmittel ist auf folgende Weise erhalten worden: 11,7 g 3-Carbäthoxy-7-amino-cumarin werden in 200 ml Eisessig gelöst, bei 20° C mit 30 ml 37%iger Salzsäure versetzt und bei 0 bis 5° C mit einer Lösung von 3,5 g Natriumnitrit in 10 ml Wasser diazotiert. Nach 30 Minuten gibt man eine Lösung von 15 g 3-Phenyl-4-benzylisoxazlin-5-on in 100 ml Eisessig hinzu und saugt nach 4 Stunden den gebildeten Farbstoff ab. Man wäscht ihn mit Wasser und trocknet ihn. Ausbeute 26,6 g, F. 114 – 115° C (Z.).
Der Farbstoff wird in 175 ml Xylol suspendiert und zu 150 ml 1-Dimethylamino-2-propanol, das auf 100 bis 110° C erhitzt ist, getropft. Man kocht dann noch 1/2 Stunde rückfließend und engt anschließend im Vakuum ein. Den Rückstand versetzt man mit 200 ml Methanol, saugt das abgeschiedene Material ab und löst es aus 1 l Alkohol um. Man erhält 9,6 g 7-[4-Benzyl-5-phenyl-1,2,3-triazolyl-(2)]-3-carbäthoxy-cumarin vom F. 151 – 152° C.

**Patentansprüche**

1. Cumarinverbindungen der Formel

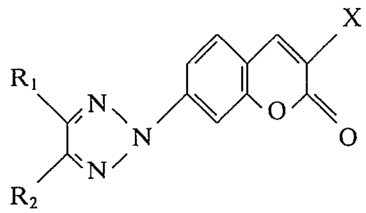

(I)

worin

$R_1$ und $R_2$ für Wasserstoff, $C_1-C_6$-Alkyl, das durch Hydroxy, $C_1-C_4$-Alkoxy, Cyan, Carboxy, Sulfo, ($C_1-C_4$-Alkoxy)-carbonyl, Amino, Mono- und Di-($C_1-C_4$-alkyl)-amino, Aminocarbonyl, Mono- und Di($C_1-C_4$-alkyl)-aminocarbonyl, Aminosulfonyl, Mono- und Di-($C_1-C_4$-alkyl)-aminosulfonyl, $C_1-C_4$-Alkylthio, Halogen, vorzugsweise Fluor, Chlor und Brom, $C_1-C_4$-Alkylsulfonyl und Phenyl substituiert sein kann;
Phenyl, das durch $C_1-C_4$-Alkyl, Trifluormethyl, Chlor, Brom, Cyan, ($C_1-C_4$-Alkoxy)-carbonyl, $C_1-C_4$-Alkoxy und Phenyl substituiert sein kann;
Phenyl-$C_1-C_4$-alkyl, das im Phenylkern noch durch Chlor, $C_1-C_4$-Alkyl und $C_1-C_4$-Alkoxy substituiert sein kann, stehen, wobei
$R_1$ und $R_2$ jedoch nicht gleichzeitig Wasserstoff bedeuten können und
X Cyan oder $-COOR_3$ bedeutet, wobei

$R_3$ für $C_1-C_6$-Alkyl, das durch Hydroxy, $C_1-C_4$-Alkoxy, Cyan, Carboxy, Sulfo, ($C_1-C_4$-Alkoxy)-carbonyl, Amino, Mono- und Di-($C_1-C_4$-alkyl)-amino, Aminocarbonyl, Mono- und Di-($C_1-C_4$-alkyl)-aminocarbonyl, Aminosulfonyl, Mono- und Di-($C_1-C_4$-alkyl)-aminosulfonyl, $C_1-C_4$-Alkylthio, Halogen, vorzugsweise Fluor, Chlor und Brom, $C_1-C_4$-Alkylsulfonyl und Phenyl substituiert sein kann, stehen.

2. Verfahren zum Weißtönen von organischen hochmolekularen Materialien, dadurch gekennzeichnet, daß man eine Verbindung gemäß Anspruch 1 verwendet.

## Claims

1. Coumarin compounds of the formula

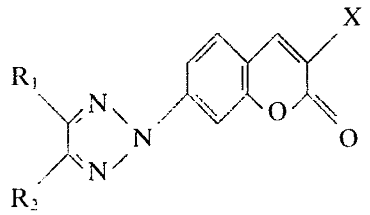

(I)

in which

$R_1$ and $R_2$ represent hydrogen; $C_1-C_6$-alkyl which can be substituted by hydroxyl, $C_1-C_4$-alkoxy, cyano, carboxyl, sulpho, ($C_1-C_4$-alkoxy)-carbonyl; amino, mono- and di-($C_1-C_4$-alkyl)-amino, aminocarbonyl, mono- and di-($C_1-C_4$-alkyl)-aminocarbonyl, aminosulphonyl, mono- and di-($C_1-C_4$-alkyl)-aminosulphonyl, $C_1-C_4$-alkylthio, halogen, preferably fluorine, chlorine, and bromine, $C_1-C_4$ alkylsulphonyl and phenyl; phenyl which can be substituted by $C_1-C_4$-alkyl, trifluoromethyl, chlorine, bromine, cyano, ($C_1-C_4$-alkoxy)-carbonyl, $C_1-C_4$-alkoxy and phenyl; or phenyl$-C_1-C_4$-alkyl which can also be substituted in the phenyl nucleus by chlorine, $C_1-C_4$-alkyl and $C_1-C_4$-alkoxy, but $R_1$ and $R_2$ cannot simultaneously designate hydrogen, and

X designates cyano or $-COOR_3$,

wherein

$R_3$ represents $C_1-C_6$-alkyl which can be substituted by hydroxyl, $C_1-C_4$-alkoxy, cyano, carboxyl, sulpho, ($C_1-C_4$-alkoxy)-carbonyl, amino, mono- and di-($C_1-C_4$-alkyl)-amino, aminocarbonyl, mono- and di-($C_1-C_4$-alkyl)-aminocarbonyl, aminosulphonyl, mono- and di-($C_1-C_4$-alkyl)-aminosulphonyl, $C_1-C_4$-alkylthio, halogen, preferably fluorine, chlorine and bromine, $C_1-C_4$-alkylsulphonyl and phenyl.

2. Process for whitening organic high-molecular materials, characterised in that a compound according to Claim 1 is used.

## Revendications

1. Des composés de coumarine de formule:

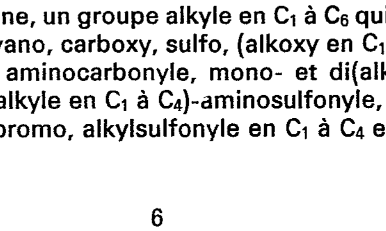

(I)

dans laquelle

$R_1$ et $R_2$ représentent de l'hydrogène, un groupe alkyle en $C_1$ à $C_6$ qui peut être substitué par un radical hydroxy, alkoxy en $C_1$ à $C_4$, cyano, carboxy, sulfo, (alkoxy en $C_1$ à $C_4$)-carbonyle, amino, mono- et di(alkyle en $C_1$ à $C_4$)-amino, aminocarbonyle, mono- et di(alkyle en $C_1$ à $C_4$)-aminocarbonyle, aminosulfonyle, mono- et di(alkyle en $C_1$ à $C_4$)-aminosulfonyle, alkylthio en $C_1$ à $C_4$, halogéno, de préférence fluoro, chloro et bromo, alkylsulfonyle en $C_1$ à $C_4$ et phényle; un groupe phényle qui

peut être substitué par un radical alkyle en $C_1$ à $C_4$, trifluorométhyle, chloro, bromo, cyano, (alkoxy en $C_1$ à $C_4$)-carbonyle, alkoxy en $C_1$ à $C_4$ et phényle;
un groupe phényl-alkyle en $C_1$ à $C_4$ qui peut encore être substitué dans le noyau phényle par du chlore, un radical alkyle en $C_1$ à $C_4$ et un radical alkoxy en $C_1$ à $C_4$,

$R_1$ et $R_2$ ne pouvant toutefois pas désigner en même temps de l'hydrogène et
X est un groupe cyano ou $-COOR_3$ dans lequel
$R_3$ est un groupe alkyle en $C_1$ à $C_6$ qui peut être substitué par un radical hydroxy, alkoxy en $C_1$ à $C_4$, cyano, carboxy, sulfo, (alkoxy en $C_1$ à $C_4$)-carbonyle, amino, mono- et di-(alkyle en $C_1$ à $C_4$)-amino, aminocarbonyle, mono-et di-(alkyle en $C_1$ à $C_4$)-aminocarbonyle, aminosulfonyle, mono- et di-(alkyle en $C_1$ à $C_4$)-aminosulfonyle, alkylthio en $C_1$ à $C_4$, halogéno, de préférence fluoro, chloro et bromo, alkylsulfonyle en $C_1$ à $C_4$ et phényle.

2. Procédé d'azurage optique de matières organiques de haut poids moléculaire, caractérisé en ce qu'il consiste à utiliser un composé suivant la revendication 1.